# EUROPÄISCHE PATENTSCHRIFT

(11) **EP 1 091 697 B1**
(45) Veröffentlichungstag und Bekanntmachung des Hinweises auf die Patenterteilung: **28.04.2004**
(21) Anmeldenummer: 99959107.6
(22) Anmeldetag: 21.05.1999
(51) Int. Cl.: A61B 17/16

(54) **CHIRURGISCHES BOHRGERÄT ZUR PERFORATION DES SCHÄDELKNOCHENS**
SURGICAL DRILLING DEVICE FOR PERFORATING THE CRANIUM
APPAREIL DE PER AGE CHIRURGICAL POUR LA PERFORATION DU CRANE

(30) Priorität: 01.07.1998 DE 19829406
(43) Veröffentlichungstag der Anmeldung: 18.04.2001
(73) Patentinhaber: Aesculap AG & Co. KG, 78532 Tuttlingen (DE)
(72) Erfinder: KUPFERSCHMID, Bernhard, D-78576 Emmingen-Liptingen (DE); WEISSHAUPT, Dieter, D-78194 Immendingen (DE); LERCH, Karl-Dieter, D-58452 Witten (DE)
(74) Vertreter: Böhme, Ulrich, Dr. Dipl.-Phys.
(86) Internationale Anmeldenummer: PCT/EP1999/003496
(87) Internationale Veröffentlichungsnummer: WO 2000/001309

(56) Entgegenhaltungen:
- DE-B- 2 916 221
- DE-U- 29 811 640

## Beschreibung

Die Erfindung betrifft ein chirurgisches Bohrgerät zur Perforation des Schädelknochens mit einer auf dem Schädelknochen aufsetzbaren Bohrlehre und einem in der Bohrlehre geführten Bohrer.

Derartige Bohrgeräte werden benötigt, um beispielsweise ein Ventrikel-Drainagesystem zur Druckentlastung des Schädelinneren installieren zu können. Dabei ist es bekannt, Bohrlehren zu verwenden, durch die ein mit einem Handstück versehener Bohrer eingeführt wird (US-A 4 821 716). Der Bohrer wird dabei von Hand gegen den Schädelknochen gedrückt und solange gedreht, bis der Schädel durchbohrt ist. Dabei besteht die Gefahr, daß durch unsachgemäße Druckbeaufschlagung des Handbohrers vermeidbare Verletzungen erzeugt werden.

Es ist Aufgabe der Erfindung, ein gattungsgemäßes chirurgisches Bohrgerät so auszugestalten, daß mit ihm Verletzungen beim Bohren soweit wie möglich vermieden werden.

Diese Aufgabe wird bei einem chirurgischen Bohrgerät der eingangs beschriebenen Art erfindungsgemäß dadurch gelöst, daß der Bohrer in einer in der Bohrlehre drehbar gelagerten Halterung in Richtung der Drehachse verschiebbar und relativ zur Halterung drehfest aufgenommen ist und daß der Bohrer gegen die Wirkung einer Feder in Richtung auf die Halterung verschiebbar ist.

Bei einer solchen Ausgestaltung wird also der Bohrer mit einer definierten Federkraft gegen den Schädelknochen gedrückt, so daß die axial auf den Bohrer wirkende Kraft unabhängig davon ist, wie stark der Benutzer die Bohrlehre und eine Antriebsvorrichtung für den Bohrer gegen den Schädelknochen drückt. Die Andruckkraft des Bohrers wird allein von der Feder bestimmt und ist daher so wählbar, daß eine möglichst geringe Verletzung des unter dem Schädelknochen angeordneten Gehirngewebes gewährleistet wird.

Günstig ist es, wenn ein Anschlag vorgesehen ist, der die Verschiebung des Bohrers unter der Wirkung der Feder relativ zu der Halterung begrenzt. Dadurch ist sichergestellt, daß auch vor dem Aufsetzen des Bohrers auf den Schädelknochen der Bohrer in axialer Richtung an der Halterung unverlierbar gehalten ist.

Weiterhin ist es vorteilhaft, wenn der Bohrer einen die Bohrtiefe begrenzenden Anschlag trägt, beispielsweise einen auf der Oberseite des Schädelknochens auflegbaren Flansch. Dieser Anschlag begrenzt die Eindringtiefe des Bohrers in den Schädelknochen und kann so gewählt sein, daß der Schädelknochen gerade durchbohrt wird, ohne daß der Bohrer wesentlich in das darunterliegende Gehirngewebe eintritt und dort Verletzungen verursacht.

Bei einer bevorzugten Ausfertigung weist die Halterung ein Sackloch zur Aufnahme des Bohrers auf. Man erhält dadurch eine sehr kompakte Konstruktion. Dabei ist es günstig, wenn in dem Sackloch eine den Bohrer aus diesem ausschiebende Feder angeordnet ist, also die Feder, die den Bohrer mit der gewünschten Federkraft gegen den Schädelknochen drückt.

Der Antrieb des Bohrers kann in verschiedener Weise erfolgen, gegebenenfalls maschinell, in den meisten Fällen wird jedoch der Antrieb manuell vorgenommen werden.

Dazu kann beispielsweise vorgesehen sein, daß die Halterung mit einer Schraubenführung für ein längsverschieblich auf dieser gelagertes, in die Schraubenführung eingreifendes Handstück versehen ist. Ein solches Handstück kann längs der Schraubenführung auf und ab bewegt werden und verdreht dadurch die Halterung und damit auch den Bohrer.

Bei einer anderen Ausgestaltung ist vorgesehen, daß die Halterung ein Zahnrad trägt, welches direkt oder indirekt mit einem an der Bohrlehre drehbar gelagerten, mittels eines Griffelementes verdrehbaren Zahnkranz im Eingriff steht.

Dabei ist es günstig, wenn der Zahnkranz an der Innenseite eines die Bohrlehre überfangenden, haubenförmigen Griffelementes angeordnet ist. Damit erhält man einen sehr kompakten Aufbau einer solchen Bohrlehre, die damit auch über einen Antrieb für den Bohrer verfügt.

Die Bohrlehre kann beispielsweise zylinderförmig ausgebildet sein und Standfüße zum Aufsetzen auf den Schädelknochen aufweisen, insbesondere sind dabei drei Standfüße vorgesehen.

Gemäß einer bevorzugten Ausführungsform ist vorgesehen, daß die Bohrlehre zur Positionierung auf dem Schädelknochen längs ihres Umfanges ein Markierungselement trägt. Dieses Markierungselement ermöglicht es dem Chirurgen, die Bohrlehre auf dem Schädelknochen in einer ganz bestimmten Position aufzusetzen, so daß sichergestellt ist, daß die Bohrung an der gewünschten Stelle erfolgt.

Insbesondere kann das Markierungselement zwei rechtwinklige Begrenzungslinien aufweisen. Diese Begrenzungslinien lassen sich an entsprechenden Markierungslinien, die der Chirurg auf dem Schädelknochen anbringt, anlegen, beispielsweise können diese Markierungslinien Verbindungslinien von Ohr zu Ohr einerseits und rechtwinklig dazu verlaufende Verbindungslinien der Nasenwurzel mit der Mitte des Hinterkopfes sein. Wenn das rechtwinklige Markierungselement mit der Ecke genau an den Kreuzungspunkt der beiden Linien angelegt wird und die Begrenzungslinien genau parallel zu den Verbindungslinien verlaufen, ist die exakte Lage der Bohrlehre auf dem Schädelknochen definiert.

Es wäre auch möglich, daß die Bohrlehre einen seitlichen Vorsprung mit rechtwinklig zueinander verlaufenden Seitenflächen aufweist und daß die Seitenflächen tangential in die Außenkontur der Bohrlehre übergehen. Dies kann über die gesamte Gehäusehöhe der Bohrlehre erfolgen, es kann aber auch vorgesehen sein, daß der Vorsprung nur im Bereich eines schädelseitigen Flansches der Bohrlehre vorgesehen ist.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht einer auf den Schädelknochen aufgesetzten Bohrlehre mit einem federnd einschiebbaren Bohrer bei Beginn der Bohrung;
- Figur 2:: eine Schnittansicht längs Linie 2-2 in Figur 1;
- Figur 3:: eine Draufsicht auf den Bohrer der Figur 1 in Richtung des Pfeiles A;
- Figur 4:: eine Schnittansicht längs Linie 4-4 in Figur 1 mit dem Bohrer in einer Stellung nach Beendigung des Bohrvorganges und
- Figur 5:: eine Ansicht ähnlich Figur 1 bei einem anderen bevorzugten Ausführungsbeispiel eines chirurgischen Bohrers.

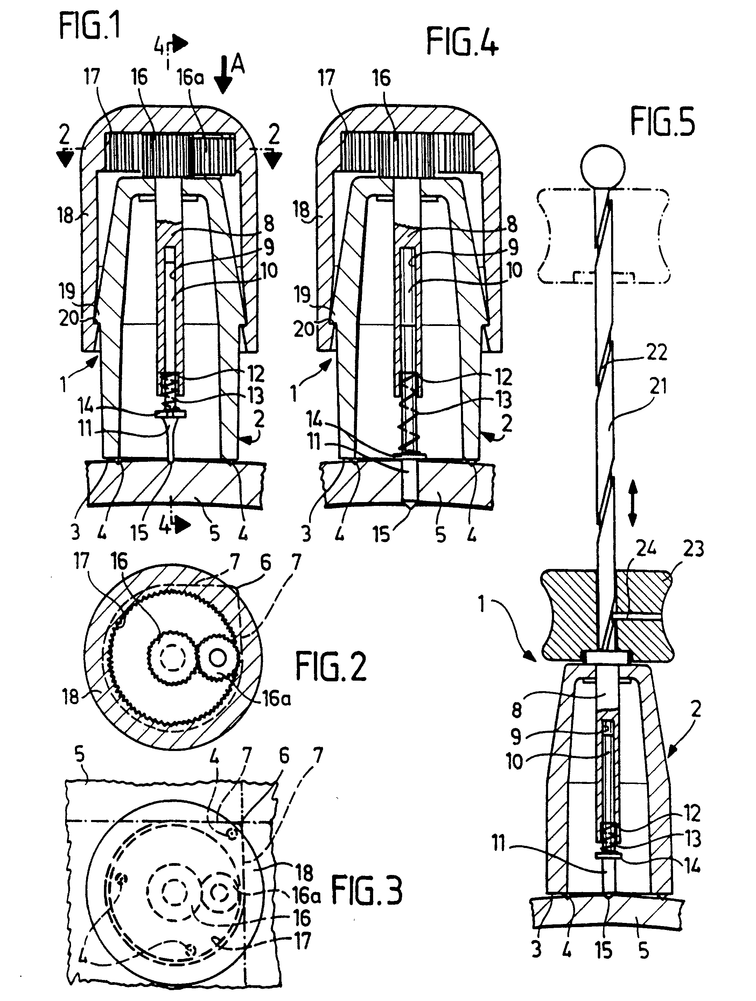

Das in den Figuren 1 bis 4 in der Zeichnung dargestellte Bohrgerät 1 umfaßt ein topfförmiges Gehäuse 2, das im Bereich seines unteren Randes 3 über den Umfang verteilt drei spitze Vorsprünge 4 trägt, mit denen das Gehäuse 2 auf einem Schädelknochen 5 festgelegt werden kann. Das Gehäuse 2 hat einen kreisförmigen Querschnitt, allerdings springt es an einer Seite in Form eines Vorsprunges 6 vor, der durch rechtwinklig zueinander verlaufende Seitenflächen 7 begrenzt ist, die tangential in den Umfang des zylindrischen Teiles des Gehäuses 2 einmünden. Damit erhält man im Bereich des Randes 3 einen Querschnitt, der über 270° kreisförmig ist, über den restlichen Umfangsbereich dagegen rechtwinklig vorspringt. Dieser Vorsprung 6 dient als Markierungselement, so daß man beispielsweise durch Anlage der Seitenflächen 7 an rechtwinklige Markierungslinien auf dem Schädelknochen das Gehäuse auf demselben exakt positionieren kann (Fig. 3).

In dem an der Oberseite geschlossenen Gehäuse 2 ist eine stabförmige Halterung 8 um die Längsachse des Gehäuses 2 drehbar und axial unverschieblich gelagert, diese Halterung 8 weist ein zum unteren Ende offenes, zentrales Sackloch 9 auf, in welches der Schaft 10 eines Bohrers 11 eintaucht. Der Schaft 10 ist in dem Sackloch 9 axial frei verschiebbar aufgenommen, jedoch ist der Schaft 10 gegenüber der Halterung 8 nicht verdrehbar. Dies läßt sich durch entsprechende Formgebung des Querschnittes des Schaftes 10 und des Sackloches 9 erreichen oder durch geeignete Drehmitnehmer.

An einer Stufe 12 des Sackloches 9 stützt sich eine den Schaft 10 umgebende Schraubenfeder 13 ab, deren anderes Ende an einem Ringflansch 14 am Bohrer 11 anliegt. Dieser Ringflansch 14 bildet gleichzeitig einen Tiefenanschlag für den Bohrer 11, die Länge des Bohrers 11 von seiner Spitze 15 bis zu dem Ringflansch 14 entspricht im wesentlichen der Dicke des zu bohrenden Schädelknochens 5.

Damit der Schaft 10 nicht aus dem Sackloch 9 herausfällt, ist ein entsprechender Anschlag vorgesehen, der in der Zeichnung jedoch nicht dargestellt ist.

Die Halterung 8 ragt nach oben aus dem Gehäuse 2 heraus und trägt dort ein Ritzel 16, welches mit einem weiteren auf dem Gehäuse 2 um eine parallele Drehachse verdrehbaren Ritzel 16a kämmt.

Dieses wiederum greift in einen Innenzahnkranz 17 einer das Gehäuse 2 an der Oberseite überfangenden und um die Mittelachse des Gehäuses 2 drehbaren Haube 18 ein, die auf dem Gehäuse 2 in axialer Richtung festgelegt ist, beispielsweise durch eine Rastverbindung 19, 20.

Verdreht man die Haube 18 relativ zum Gehäuse 2, so überträgt sich diese Drehverbindung über die beiden Ritzel 16a und 16 auf die Halterung 8 und damit auf den Bohrer 11.

Zur Herstellung einer Bohrung wird der Bohrer 11 mit seinem rechtwinkligen Vorsprung 6 in der gewünschten Weise an Markierungslinien auf der Oberseite des Schädelknochens angelegt und dadurch exakt positioniert. Durch einen axialen Druck auf das Gehäuse 2 graben sich die spitzen Vorsprünge 6 geringfügig in den Schädelknochen 5 ein und fixieren somit die Position des Gehäuses 2. Der Bohrer 11 liegt dabei unter der Wirkung der Schraubenfeder 13 mit seiner Spitze 15 an der Außenseite des Schädelknochens 5 an und gräbt sich bei Verdrehung des Bohrers 11 unter Ausbildung einer Bohrung in den Schädelknochen 5 ein. Die Vorschubbewegung wird dabei ausschließlich durch die Schraubenfeder 13 bestimmt, bis der Ringflansch 14 an der Außenseite des Schädelknochens 5 zur Anlage kommt und damit die Eintauchtiefe des Bohrers 11 beschränkt. Auf diese Weise wird der Schädelknochen 5 genau durchbohrt, ohne daß darunterliegendes Gehirngewebe beschädigt wird.

Das Ausführungsbeispiel der Figur 5 gleicht dem der Figuren 1 bis 4 weitgehend, gleiche Teile tragen daher dieselben Bezugszeichen.

Im Unterschied zum Ausführungsbeispiel der Figuren 1 bis 4 ist hier ein anderes Antriebssystem für die Halterung 8 vorgesehen, es fehlen daher die Haube 18 mit Innenzahnkranz 17 sowie die beiden Ritzel 16 und 16a. Bei dieser Ausführungsform ist die Halterung 8 vielmehr in Form eines langen Schaftes 21 nach außen verlängert, der eine steile Schraubennut 22 aufweist und auf dem ein ringförmiges Griffelement 23 höhenverschiebbar gelagert ist. Dieses greift mit einem Vorsprung 24 in die Schraubennut 22 ein und verdreht dadurch bei seiner Höhenverschiebung den Schaft 21. Durch die Höhenverschiebung des Griffelementes 23 kann somit der Bohrer 11 in der gewünschten Weise verdreht werden.

## Patentansprüche

1. Chirurgisches Bohrgerät zur Perforation des Schädelknochens mit einer auf dem Schädelknochen aufsetzbaren Bohrlehre (2) und einem in der Bohrlehre (2) geführten Bohrer (10, 11), **dadurch gekennzeichnet, daß** der Bohrer (10, 11) in einer in der Bohrlehre (2) drehbar gelagerten Halterung (8) in Richtung der Drehachse verschiebbar und relativ zur Halterung (8) drehfest aufgenommen ist und daß der Bohrer (10, 11) gegen die Wirkung einer Feder (13) in Richtung auf die Halterung (8) verschiebbar ist.

2. Bohrgerät nach Anspruch 1, **dadurch gekennzeichnet, daß** ein Anschlag vorgesehen ist, der die Verschiebung des Bohrers unter der Wirkung der Feder (13) relativ zu der Halterung (8) begrenzt.

3. Bohrgerät nach Anspruch 2, **dadurch gekennzeichnet, daß** der Bohrer (11) einen die Bohrtiefe begrenzenden Anschlag (14) trägt.

4. Bohrgerät nach einem der voranstehenden Ansprüche, **dadurch gekennzeichnet, daß** die Halterung (8) ein Sackloch (9) zur Aufnahme des Bohrers (10, 11) aufweist.

5. Bohrgerät nach Anspruch 4, **dadurch gekennzeichnet, daß** in dem Sackloch (9) eine den Bohrer (10, 11) aus diesem ausschiebende Feder (13) angeordnet ist.

6. Bohrgerät nach einem der voranstehenden Ansprüche, **dadurch gekennzeichnet, daß** die Halterung (8) mit einer Schraubenführung (22) für ein längsverschieblich auf dieser gelagertes, in die Schraubenführung (22) eingreifendes Handstück (23) versehen ist.

7. Bohrgerät nach einem der Ansprüche 1 bis 5, **dadurch gekennzeichnet, daß** die Halterung (8) ein Zahnrad (16, 16a) trägt, welches mit einem an der Bohrlehre (2) drehbar gelagerten, mittels eines Griffelementes (18) verdrehbaren Zahnkranz (17) kämmt.

8. Bohrgerät nach Anspruch 7, **dadurch gekennzeichnet, daß** der Zahnkranz (17) auf der Innenseite eines die Bohrlehre (2) überfangenden, haubenförmigen Griffelementes (18) angeordnet ist.

9. Bohrgerät nach einem der voranstehenden Ansprüche, **dadurch gekennzeichnet, daß** die Bohrlehre (2) zylinderförmig ausgebildet ist, und Standfüße (4) zum Aufsetzen auf dem Schädelknochen (5) aufweist.

10. Bohrgerät nach Anspruch 9, **dadurch gekennzeichnet, daß** drei Standfüße (4) vorgesehen sind.

11. Bohrgerät nach einem der voranstehenden Ansprüche, **dadurch gekennzeichnet, daß** die Bohrlehre (2) zur Positionierung auf dem Schädelknochen (5) längs ihres Umfanges ein Markierungselement (6) trägt.

12. Bohrgerät nach Anspruch 11, **dadurch gekennzeichnet, daß** das Markierungselement (6) zwei rechtwinklige Begrenzungslinien (7) aufweist.

13. Bohrgerät nach Anspruch 12, **dadurch gekennzeichnet, daß** die Bohrlehre (2) einen seitlichen Vorsprung (6) mit rechtwinklig zueinander verlaufenden Seitenflächen (7) aufweist und daß die Seitenflächen (7) tangential in die Außenkontur der Bohrlehre (2) übergehen.

14. Bohrgerät nach Anspruch 13, **dadurch gekennzeichnet, daß** der Vorsprung (6) nur im Bereich eines schädelseitigen Flansches des Bohrlehre (2) vorgesehen ist.

## Claims

1. A surgical drilling device for perforating cranial bone, having a jig (2) placeable on the cranial bone and a drill (10, 11) guided within the jig (2), **characterised in that** the drill (10, 11) is accommodated in a holding device (8) so as to be displaceable in the direction of the axis of rotation and non-rotatable relative to the holding device (8), the latter being rotatably mounted in the jig, and **in that** the drill (10, 11) is displaceable against the action of a spring (13) in the direction of the holding device (8).

2. A drilling device according to Claim 1, **characterised in that** a stop which is provided limits the displacement of the drill relative to the holding device (8) under the action of the spring (13).

3. A drilling device according to Claim 2, **characterised in that** the drill (11) bears a stop (14) which limits the drilling depth.

4. A drilling device according to one of the preceding claims, **characterised in that** the holding device (8) has a blind bore (9) for accommodating the drill (10, 11).

5. A drilling device according to Claim 4, **characterised in that** a spring (13) arranged within the blind bore (9) forces the drill (10, 11) out of the said bore.

6. A drilling device according to one of the preceding claims, **characterised in that** the holding device is provided with a screw guide (22) for a handpiece (23) mounted longitudinally displaceably on the screw guide (22) and engaging therein.

7. A drilling device according to one of Claims 1 to 5, **characterised in that** the holding device (8) bears a gearwheel (16, 16a) which engages with a gear ring (17) which is mounted rotatably on the jig (2) and which may be twisted by means of a gripping member (18).

8. A drilling device according to Claim 7, **characterised in that** the gear ring (17) is arranged on the inside of a cap-like gripping member (18) covering the jig.

9. A drilling device according to one of the preceding claims, **characterised in that** the jig (2) is of a cylindrical design and has feet (4) for the purpose of placing it on the cranial bone (5).

10. A drilling device according to Claim 9, **characterised in that** three feet (4) are provided.

11. A drilling device according to one of the preceding claims, **characterised in that** the jig (2) bears a marker (6) around its circumference for the purpose of positioning it on the cranial bone (5).

12. A drilling device according to Claim 11, **characterised in that** the marker (6) has two right-angled delimiting lines (7).

13. A drilling device according to Claim 12, **characterised in that** the jig (2) has a lateral projection (6) with sides (7) extending at right angles to one another, and **in that** the sides (7) tangentially undergo a transition to form the outside contour of the jig (2).

14. A drilling device according to Claim 13, **characterised in that** the projection (6) is provided only in the region of a cranial-side flange of the jig (2).

## Revendications

1. Appareil de perçage chirurgical pour la perforation des os du crâne ou calotte crânienne, comprenant un calibre ou gabarit de perçage (2) pouvant être appliqué sur l'os du crâne, et un foret (10, 11) guidé dans le calibre de perçage (2),
**caractérisé en ce que** le foret (10, 11) est logé dans un support de maintien (8) monté rotatif dans le calibre ou gabarit de perçage (2), le foret pouvant coulisser dans le support de maintien dans la direction de l'axe de rotation et étant fixe en rotation par rapport au support de maintien (8), et **en ce que** le foret (10, 11) peut coulisser à l'encontre de l'action d'un ressort (13), en direction du support de maintien (8).

2. Appareil de perçage selon la revendication 1, **caractérisé en ce qu'**il est prévu une butée qui limite le coulissement du foret sous l'action du ressort (13), par rapport au support de maintien (8).

3. Appareil de perçage selon la revendication 2, **caractérisé en ce que** le foret (11) porte une butée (14) limitant la profondeur de perçage.

4. Appareil de perçage selon l'une des revendications précédentes, **caractérisé en ce que** le support de maintien (8) comporte un trou borgne (9) pour recevoir le foret (10, 11).

5. Appareil de perçage selon la revendication 4, **caractérisé en ce que** dans le trou borgne (9) est disposé un ressort (13) repoussant le foret (10, 11) hors de ce trou borgne.

6. Appareil de perçage selon l'une des revendications précédentes, **caractérisé en ce que** le support de maintien (8) est pourvu d'un guidage hélicoïdal (22) pour une poignée (23) qui y est montée en coulissement longitudinal et s'engage dans ce guidage hélicoïdal (22).

7. Appareil de perçage selon l'une des revendications 1 à 5, **caractérisé en ce que** le support de maintien (8) porte un engrenage ou système de roue dentée (16, 16a) qui engrène avec une couronne dentée (17) montée rotative sur le calibre ou gabarit de perçage (2) et pouvant être mise en rotation au moyen d'un élément de préhension (18).

8. Appareil de perçage selon la revendication 7, **caractérisé en ce que** la couronne dentée (17) est disposée sur le côté intérieur d'un élément de préhension (18) en forme de chapeau, qui surmonte le calibre ou gabarit de perçage (2).

9. Appareil de perçage selon l'une des revendications précédentes, **caractérisé en ce que** le calibre ou gabarit de perçage (2) est d'une configuration cylindrique et présente des pieds d'appui (4) pour être appliqué sur l'os du crâne (5).

10. Appareil de perçage selon la revendication 9, **caractérisé en ce que** sont prévus trois pieds d'appui (4).

11. Appareil de perçage selon l'une des revendications précédentes, **caractérisé en ce que** le calibre ou gabarit de perçage (2) porte sur sa périphérie, un élément de repérage (6), pour son positionnement sur l'os du crâne(5).

12. Appareil de perçage selon la revendication 11, **caractérisé en ce que** l'élément de repérage (6) présente deux lignes de délimitation (7) perpendiculaires.

13. Appareil de perçage selon la revendication 12, **caractérisé en ce que** le calibre ou gabarit de perçage (2) comporte une protubérance latérale (6) avec des surfaces latérales (7) s'étendant perpendiculairement l'une à l'autre, et **en ce que** les surfaces latérales (7) se raccordent tangentiellement au contour extérieur du calibre ou gabarit de perçage (2).

14. Appareil de perçage selon la revendication 13, **caractérisé en ce que** la protubérance (6) n'est prévue que dans la zone d'un flasque du calibre ou gabarit de perçage (2), se situant au voisinage de l'os du crâne.
